# EUROPEAN PATENT APPLICATION

(11) **EP 2 557 422 A2**
(43) Date of publication of application: **13.02.2013**
(21) Application number: 12179502.5
(22) Date of filing: 07.08.2012
(51) Int. Cl.: G01N 33/50, G01N 33/542, G01N 33/567, G01N 33/58

(54) **Device, system and method for detecting a sensory perception**

(30) Priority: 10.08.2011 DE 102011080772
(71) Applicant: Fraunhofer-Gesellschaft zur Förderung der angewandten Forschung e.V., 80686 München (DE)
(72) Inventor: Bock, Karlheinz, 82239 Alling (DE); Mohr, Gerhard, 93047 Regensburg (DE); Endres, Hanns-Erik, 80689 München (DE)
(74) Representative: Zimmermann, Tankred Klaus

(57) **Abstract**

A device for detecting a sensory perception with regard to an analyte, having a sensor system and an organic receptor cell for detecting the sensory perception. The organic receptor cell is designed to assume a cellular state, which depends on the analyte in contact with the receptor cell. The sensor system is designed to determine the cellular state and to output it to the cellular state signal, which indicates the cellular state.

## Description

The present invention relates to a device, a system and a method for detecting a sensory perception. The present invention relates in particular to a device which detects the sensory perception by determining the cellular state of an organic receptor cell.

The human or animal sensory perception of "taste" is one of the most important organs of perception of a person or an animal. The human sense of taste is based on signals from approx. 2000 taste buds, i.e., receptor cells distributed on the tongue. For example, a person's sense of taste is used to evaluate the quality and enjoyment value of foodstuffs and to detect substances, in particular hazardous substances such as cleaning agents (bitter taste). The taste perception and in particular the technical taste perception by means of a sensor system is therefore an important area of application having a number of possible applications.

In previous methods and/or devices for detecting the "taste" of an analyte, a distinction is made between two different approaches: all the measurement systems available today are based either on direct chemical analysis of the components of the analyte or on chemical and/or biochemical sensors. The ASTREE "electronic tongue" is a system that is available on the market and is based on chemical sensors used in a multimodal fashion (simultaneous use of multiple sensors). With chemical sensors, taste is detected by detecting a chemical analytical value (e.g., an electrochemical potential) that is assigned to a taste, where the correlation between the analytical value and the taste is determined with the help of so-called human panels. The term "human panel" is understood to refer to the investigation of analytes by means of taste tests by human test subjects, but it is complex in particular to use such human panels and to perform statistical analyses of the test results and these are associated with high costs.

It is the object of the present invention to provide a device and a method for detecting a sensory perception with a high precision.

The object of the present invention is achieved by a device for detecting a sensory perception with regard to an analyte according to claim 1, by a system for detecting a sensory perception with regard to an analyte according to claim 19, and by a method for detecting a sensory perception with regard to an analyte according to claim 21.

The present invention is based on the finding that a sensory perception by a human or an animal with regard to an analyte can be detected by an organic receptor cell (as an artificial organ) and by a sensor system, which picks up the cellular state and/or the reaction of the receptor cell to the analyte and converts it into an electric signal. To do so, the analyte comes in contact with the organic receptor cell. The reaction of the receptor cell of an artificial taste bud on artificial epithelium, for example, simulates or resembles the reaction of a taste bud of the organ of perception of a human or an animal and can be detected by the sensor system by means of optical, electrical or impedimetric methods, for example. The sensor system outputs a cellular state signal, which permits an inference as to the human or animal sensory perception.

According to one embodiment, the device for detecting a sensory perception with regard to an analyte comprises a sensor system and an organic receptor cell for detecting the sensory perception. The organic receptor cell is designed to assume a cellular state, which depends on the analyte coming in contact with the receptor cell. The sensor system is designed to determine the cellular state and/or to observe the reaction to the analyte and to output a cellular state signal which indicates the cellular state and can be analyzed. One advantage of this embodiment is that the detection method used is based on an organic receptor cell which is the same as or comparable to the receptor cells of the human or animal organ of perception and/or the tongue, so that it improves the comparability of the sensory perception as determined technically to the human or animal sensory perception, and this then leads to an increase in accuracy.

According to another embodiment, the device has at least one nanoparticle coupled to the organic receptor cell. The nanoparticle has a fluorescence characteristic which depends on the cellular state or optionally has an indicator dye having a florescence characteristic, wherein the cellular state depends on an ionic concentration in the receptor cell, a molecular concentration in the receptor cell, an electric potential on the receptor cell and/or an electric potential of the receptor cell, for example.

In this embodiment, the fluorescence characteristic of the nanoparticle is detected by an optical sensor of the sensor system. According to other embodiments, the optical detection may be performed by using a microtiter plate reader, a surface plasmon resonance array (SPR array), a fluorescence microscope, a Raman spectroscope, an optical detector in combination with a light source or a combination of any of these. This embodiment is advantageous in that the nanoparticle converts the information about the cellular state and/or the change in cellular state directly into an analyzable optical signal, namely the fluorescence characteristic.

According to other embodiments, the sensor system may have additional sensors for detecting the conductivity and/or for detecting an electric potential of the receptor cell by means of an electrochemical microelectrode that is electrically coupled to the receptor cell. It is advantageous that individual parameters of the cellular state, such as the electric nerve potential, can be determined directly here.

Another embodiment of the present invention comprises the device for detecting the sensory perception and a signal analyzer designed to determine the sensory perception with regard to the analyte based on the cellular state signal, where the determination can be performed by comparing the prevailing cellular state signal with a previously determined cellular state signal of a known analyte. It is advantageous here that the signal analyzer directly outputs information about the taste of the analyte (e.g., salty).

Another embodiment provides a method for detecting a sensory perception, wherein the method comprises the following steps: providing the analyte, supplying the analyte to an organic receptor cell for detecting a sensory perception, determining the cellular state of the receptor cell, which depends on the analyte in contact with the receptor cell, and output of a cellular state signal indicating the cellular state.

In the following, embodiments of the invention are explained in more detail with reference to the accompanying drawings, in which:
- Figure 1: shows a schematic diagram of a device for detecting a sensory perception according to one embodiment;
- Figure 2: shows a schematic sectional diagram of an organic receptor cell for detecting a sensory perception according to one embodiment;
- Figure 3: shows a schematic diagram of a nanoparticle according to one embodiment and
- Figure 4: shows a schematic diagram of a device for detecting a sensory perception on a substrate according to one embodiment.

The measurement systems described above, which are based on chemical analysis or chemical and/or biochemical sensors, are comparable to the functionality of human or animal taste perception only to a limited extent, so that makes it difficult to establish a correlation between the taste and the chemical analytical value. The background for this is that normally only a small group or subset of taste substances can be measured and identified by the methods of chemical analysis. In the case of chemical sensors with which the sensitivity is usually lower than with chemical analytical methods, it is possible to apply the measured value and/or analytical value to the human or animal sensory perception only to a limited extent because there is little or no comparability of the technical detection method to the "detection method" of the organ of perception of a human or animal.

Because of these restrictions in the measurement technology, there is a demand for improving the detection of a sensory perception with regard to accuracy and comparability in particular.

Before describing the present invention in greater detail below on the basis of the drawings, it should be pointed out that identical elements and structures or those having the same or similar functions in the various figures are labeled with the same reference notation, so that the description of the elements and structures labeled with the same reference notation in the various embodiments are interchangeable and/or can be applied to one another.

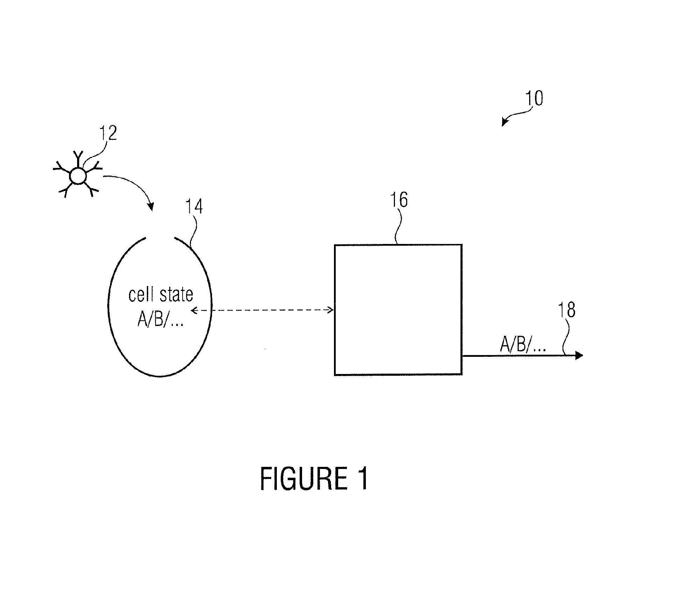

Figure 1 shows a device 10 for detecting a sensory perception with regard to an analyte 12 with an organic receptor cell 14 and a sensor system 16. The analyte 12 can come in contact with the receptor cell 14. The receptor cell 12 is designed to embody a cellular state, e.g., A or B, depending on the analyte 12 in contact with it. The sensor system 16 is connected to the receptor cell 12 to determine the cellular state of the receptor cell 12 and to output an electric cellular state signal 18 on the basis of same.

An analyte 12, e.g., a foodstuff in contact with the receptor cell 14, causes a change in the cellular state and/or a reaction of the receptor cell 14. The organic receptor cell 14 for detecting the human or animal sensory perception, for example, is designed to artificially simulate a human or animal taste bud, for example, so that the cellular state depends on the ionic concentration in the receptor cell 14, the molecular concentration of a certain molecule in the receptor cell 14, the electric potential and/or electric polarity of the receptor cell, for example. This cellular state, e.g., A at a high ionic concentration of the receptor cell 14 or B at a low ionic concentration, depending on the analyte in contact with the receptor cell, is detected optically or electrically by the sensor system 16, for example. The sensor system 16 is designed to output a cellular state signal 18, which indicates the cellular state (A or B). In this embodiment, it is advantageous that the human or animal sensory perception with regard to the analyte 12 can be converted directly into an electrical cellular state signal 18, so that such a device comes significantly closer to the human or animal sensory perception than the previous measurement systems. Examples of applications of the device 10 range from taste tests on foodstuffs and other substances such as pharmaceutical drugs to process control and toxicity tests.

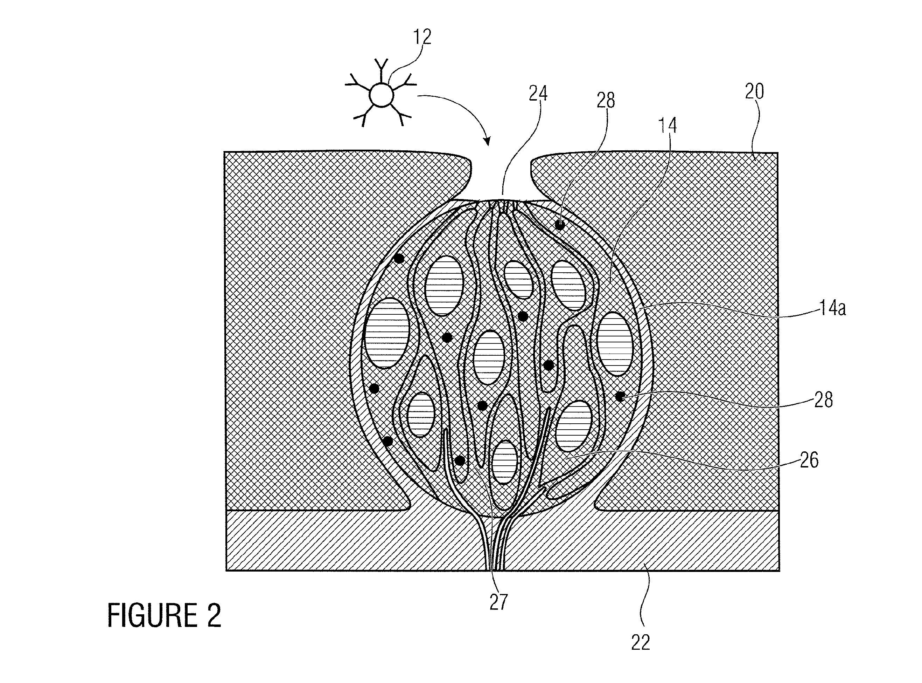

Figure 2 shows an embodiment of an oval organic receptor cell 14, which is formed on artificial epithelium 20. The artificial epithelium 20, which is arranged on a substrate 22, partially surrounds the organic receptor cell 14, wherein a cellular membrane 14a separates the epithelium 20 and the organic receptor cell 14. The cellular membrane 14a of the receptor cell 14 is not completely closed on the side opposite the substrate 22, so that a freely accessible area, namely a taste pore 24 through which it can come in contact with the analyte 12 is formed. The organic receptor cell 14 has a nerve fiber 27 and basal cells 26, each having a core extending longitudinally between the taste pore 24 and the substrate 22. In addition, the receptor cell 14 has nanoparticles 28 which are incorporated into the organic receptor cell 14 and/or are applied around the receptor cell 14 on the outside and/or are introduced into the receptor cell 14 invasively and/or noninvasively.

The synthetic organic receptor 14 simulates a human or animal taste bud with basal cells 26 and is supplied with nutrients via the artificial epithelium 20, which is a suitable environment for the receptor cell, and via the cellular membrane 14a. The receptor cell 14 is designed to undergo a change in state when it comes in contact with an analyte 12. This change in state is based on a complex mechanism of action within the receptor cell 14, in which the chemical signal of the analyte 12 is converted into a sensory perception via multiple intermediate chemical steps and can be output via the nerve fiber 27. In detection of a sensory perception, there is intervention into the chemical signal pathway at a suitable location to pick up the signals and convert them into an electronically analyzable cellular state signal. In this embodiment, the cellular state is picked up by the nanoparticles 28, which have a fluorescence characteristic that depends on the cellular state. In particular, the nanoparticles 28 respond to changes in concentration, changes in charge and/or changes in polarity of the receptor cell 14 with a fluorescence phenomenon having the fluorescence characteristic or with a change in the fluorescence characteristic. This fluorescence characteristic is defined by (and/or comprises) a fluorescence intensity, a fluorescence decay time, a fluorescence polarization and/or additional measurable properties of fluorescence. The respective fluorescence characteristic is detected via a sensory system (cf. sensor system 16, Figure 1) using an optical sensor, and thus the cellular state signal is output by the same. Possible optical sensors for this include a confocal fluorescence microscope, a high-resolution fluorescence microscope (e.g., stimulated emission depletion), conventional CCD arrays in combination with a light source (e.g., a light-emitting diode or laser diode), a microtiter plate reader, a surface plasmon array or film-printed organic thin-film transistors in combination with an electroluminescence paste.

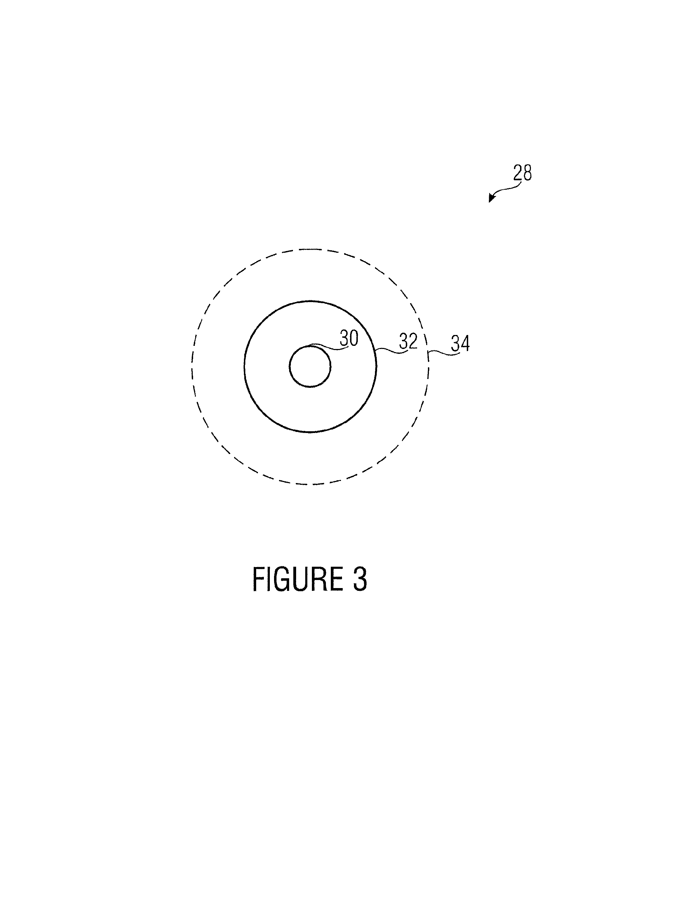

Figure 3 shows an embodiment of a nanoparticle 28 corresponding to the nanoparticles according to Figure 2. The nanoparticle 28 has a core 30 and a shell 32 surrounding the core 30. In addition, the nanoparticle 28 may have another optional shell 34 surrounding the core 30 and the shell 32.

The shell 32 has an indicator dye that responds selectively to different analyte ions and/or analyte molecules or to a change in polarity, e.g., in the particle environment, by a change in the fluorescence characteristic. The core 30 has a reference dye with a predetermined fluorescence characteristic which permits a stable and referenced measurement and does not depend on the cellular state.

The optional shell 34 has antibodies and/or ligands which enable selective specific binding of the nanoparticle 28 to predetermined regions of the receptor cell, e.g., to the cell membrane surface (cf. cell membrane 14a, Figure 2) or to predetermined molecules in the receptor cell. Selective coupling of the nanoparticles 28 is accomplished via ligand-receptor interaction or antibody-antigen interaction, so that the individual nanoparticle 28 can be coupled to functional groups at its surface and therefore a specific characteristic of the cellular state can be detected in a targeted manner. For example, the nanoparticle 28, which is introduced into the receptor cell by way of various endocytosis pathways, may come in contact with the cytosol (fluid containing ions) of the receptor cell and may react to a change in the ionic concentration there.

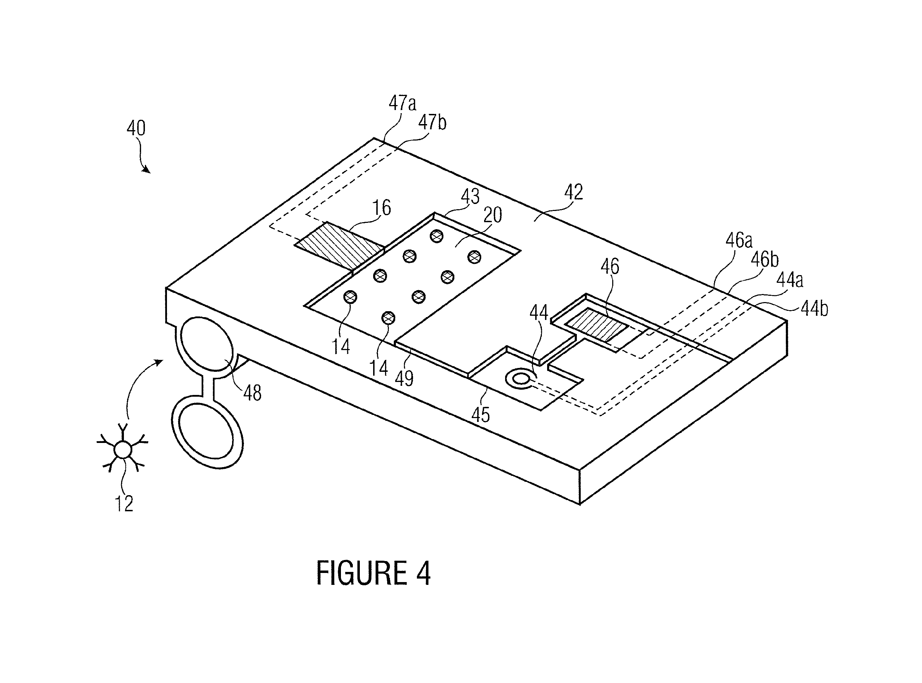

Figure 4 shows an embodiment of a device 40 for detecting a sensory perception with regard to an analyte having eight organic receptor cells. The eight organic receptor cells 14 having nanoparticles, for example, are arranged on an artificial epithelium 20. In this embodiment, a housing 42 forms the substrate. The housing 42 has a recess 43 for the artificial epithelium 20 having a channel 49 connecting the artificial epithelium 20 to another recess 45. The housing 42 additionally has the sensor system 16, which is arranged in the recess 43 and is connected to the receptor cell 14. The sensor system 16 may be implemented as an organic electronic unit on a polymer, as a silicon microelectronic unit or as a combination of these two technologies, for example, by embedding an ASIC (application-specific integrated circuit) into the polymer substrate. The sensor system 16 is electrically contacted via the connecting lines 47a and 47b. As an example, two sensors 44 and 46 for determining ambient conditions of the epithelium 20 are arranged in the additional recess 45 and can be contacted electrically via the connecting lines 44a and 44b and/or 46a and 46b. Furthermore, the housing 42 has a closable analyte receptacle 48.

The device 40 corresponds functionally to the device 10 according to Figure 1, where the eight organic receptor cells 14 each react selectively to different analyte type groups. The background for this is that each receptor cell 14 is designed like the human or animal organ of perception, to react selectively with a change in cellular state in response to different analyte type groups, e.g., the salty, sweet, acidic, bitter or umami analyte, or to differences within type groups, e.g., the intensity of the analyte of one of the type groups. These distinctions are detected by different signals of one of eight specialized receptor cells 14. In a technical sense, this embodiment corresponds to a sensor array and/or a multimodal sensor system. It is advantageous here that the different analyte type groups can be differentiated and that differences are also discernible within the type groups.

The analyte to be analyzed is supplied to the receptor cells via the analyte receptacle 48, which is effectively connected to the receptor cells 14. According to the embodiment in Figure 1, the resulting cellular state of the receptor cells 14 is determined by the sensor system 16 and is output via the connecting lines 47a and 47b, wherein the sensor system 16 either observes all the receptor cells 14 in parallel or a separate sensor system is designed for each receptor cell 14. Ambient conditions such as temperature, conductivity and redox of the epithelium 20 and thus of the organic receptor cells 14 which are cultured on the epithelium 20 and/or on the substrate 42 are determined and/or monitored by means of the sensors 44 and 46. The sensor data for determining the ambient conditions is output over the connecting lines 44a and 44b of the sensor 44 or is output over the connecting lines 46a and 46b of the sensor 46.

Alternatively, the substrate 42 may have a fluid system for supplying fluid to the epithelium 20 and/or to the organic receptor cells 14; the analytes can be brought into contact with the receptor cells 14 via the analyte receptacle 48 by means of this system. Another object of the fluid system is to maintain constant ambient conditions, e.g., the temperature for the receptor cells 14 and for the epithelium 20.

Alternatively, the electronic unit and in particular the sensor system 16 may be embodied as a planar electronic unit or an optical polymer sensor system and may be a component of the fluid system or of the substrate 42 and/or 22, so that the sensor system 16 is coupled directly to the receptor cell 14 and/or to the cell membrane 14a (cf. Figure 2).

According to other embodiments, the sensor system comprises an impedance meter and/or a conductivity meter to determine the cellular state of the receptor cell on the basis of an impedometric measurement or an impedance measurement. This conductivity meter may be arranged either directly on the receptor cell or on the epithelium. This impedance meter and/or conductivity meter can also record impedance spectra, for example.

Alternatively, the cellular state can be detected by means of a potential measurement. To do so, the sensor system has an electrochemical microelectrode, which is electrically connected to the receptor cell and is also known as an ultramicroelectrode for determining the electric potential of the receptor cell by electrochemical means. As an alternative to this, the nerve potential is determined by means of a microelectrode by connecting the microelectrode noninvasively to the nerve (and/or nerve fiber 27, Figure 2) by means of a CMOS electronic unit, for example.

It should be pointed out that the receptor cell will not necessarily have a nanoparticle for optical detection of the cellular state in embodiments in which the cellular state is determined electrically and on the basis of the nerve potential and/or impedimetrically, for example.

According to additional embodiments, the sensor system is embodied as a combination of an optical sensor, an impedance sensor and/or a sensor for determining the potential of the receptor cell and/or the nerve potential. Combining different physical measurement methods has the advantage that it prevents faulty measurements and the signal-to-noise ratio is increased, which usually increases the measurement certainty.

Although it may be assumed that in the embodiments described above, these nanoparticles react to a change in the cellular state with a change in the fluorescence characteristic, it should be pointed out that the nanoparticles may also be designed to have an absorption characteristic that depends on the cellular state. It would also be conceivable for the nanoparticles to be designed to have a UV-Vis characteristic (characteristic in the ultraviolet and visible ranges) or to have a Raman characteristic or an IR characteristic (characteristic in the infrared range) or to have an SPR characteristic (surface plasmon resonance characteristic) as a function of the cellular state. In addition, it is pointed out that alternatively, the nanoparticles may also be attached to the receptor cell or to the cell membrane surface outside of the receptor cell (cf. cell membrane 14a, Figure 2). It should also be pointed out that the receptor cell may also have different nanoparticles which react to different characteristics of the cellular state.

With reference to Figure 3, it is pointed out that alternatively, the nanoparticle may have a homogeneous structure comprised of a combination of the indicator dye and/or the reference dye instead of having a core-shell structure. The nanoparticle having a homogeneous structure may enable selective coupling to predetermined regions of the receptor cell and/or predetermined molecules, for example, by means of ligands or antibodies according to another embodiment.

An alternative embodiment of the nanoparticle 28 shown in Figure 3 has a core 30 comprised of a porous zeolite, which has an AlOₓ group or an SiOₓ group and reacts to an ion exchange with absorption. According to another embodiment, the core of the nanoparticle may consist of a metal particle and/or a magnetic particle, so that external manipulation of the nanoparticle is possible. Furthermore, according to another embodiment, use of metal nanoparticles with functional surfaces would also be conceivable (e.g., quantum dots, up-converting nanoparticles, photonic crystals, etc.). Such metal nanoparticles can be detected by means of an optical sensor, e.g., detection by means of surface-enhanced Raman spectroscopy (SERS).

With reference to Figure 2, the receptor cell 14 and/or the cellular membrane 15 of the receptor cell 14 has another embodiment of an indicator dye or marker dyes for labeling viable/dead cells, cell membranes, cell nuclei, endosomes, lysosomes, mitochondria and colored or fluorescent aptamers, peptides, proteins (e.g., green fluorescent protein), antibodies, antigens, DNA and/or RNA, so that the receptor cell 14 and/or the cellular membrane 14a will directly have an optically detectable fluorescent characteristic that depends on the cellular state of the receptor cell 14.

With reference to Figure 4, the device 40 may be part of the system for detecting a sensory perception, wherein the system also has a signal analyzer which determines the sensory perception on the basis of the cellular state signal. According to additional embodiments, the determination is performed by comparing the detected cellular state signal with cellular state signals detected previously on known analytes. Since the device 40 has a plurality of organic receptor cells 14, the signal analyzer is designed to ascertain the sensory perception from a plurality of cellular state signals and/or from a combination of different cellular state signals. The determination and/or linkage of various cellular state signals is performed by multivariant statistical methods, for example, such as main component analysis, estimation of causal models (path analysis, partial least square) or neural networks. In the technical sense, this system is to be understood as a sensor array having multimodal sensor systems. One further advantage is that different cellular state signals which provide information about the cellular state of different receptor cells can be combined by means of this signal analyzer. Alternatively, the signal analyzer may be implemented as a computer, a CPU or a program that can be executed on a CPU.

Although individual aspects of the present invention were mentioned above with respect to a device, it is pointed out here that these aspects also apply to a method for detecting a sensory perception. With reference to Figure 4, the method comprises the following steps: preparing the analyte 12; supplying the analyte 12 to the organic receptor cells 14 by means of the analyte receptacle 48; determining the cellular state of the receptor cells 14; and output of the cellular state signal indicating the cellular state.

It is pointed out that the embodiments described above for detection of a sensory perception apply to the detection of a human sensory perception as well as the detection of an animal sensory perception, for example, that of a dog.

## Claims

1. A device (10, 40) for detecting a sensory perception with regard to an analyte (12), comprising:
an organic receptor cell (14) for detecting the sensory perception, which is implemented to assume a cellular state that depends on the analyte (12) in contact with the receptor cell (14);
a sensor system (16), which is designed to determine the cellular state and to output a cellular state signal (18) indicating the cellular state; and
at least one nanoparticle (28) coupled to the organic receptor cell (14), wherein the nanoparticle (28) has a fluorescence characteristic that depends on the cellular state caused by the attached analyte (12) or comprises a UV-Vis or Raman or IR or SPR characteristic,
wherein the sensor system (16) comprises an optical sensor, which is implemented to detect the fluorescence characteristic or the UV-Vis or Raman or IR or SPR characteristic of the nanoparticle (28).

2. The device (10, 40) according to claim 1, wherein the cellular state depends on the ionic concentration in the receptor cell (14), a molecular concentration in the receptor cell, an electrical potential on the receptor cell and/or an electrical polarity of the receptor cell.

3. The device (10, 40) according to claim 1, wherein the nanoparticle (28) comprises a reference dye having a predetermined fluorescence characteristic and/or comprises an indicator dye.

4. The device (10, 40) according to claim 3, wherein the nanoparticle (28) comprises a core (30) with the reference dye and/or a shell (32) with the indicator dye.

5. The device (10, 40) according to any one of claims 1 to 4, wherein the nanoparticle (28) is implemented to be selectively coupled to or within a predetermined range of the receptor cell (14).

6. The device (10, 40) according to claim 5 in depending back on claim 4, wherein the nanoparticle (28) has an additional shell (34) around the core (30) by means of which the nanoparticle can be selectively coupled.

7. The device (10, 40) according to any one of claims 1 to 6, wherein the optical sensor comprises a microtiter plate reader, a surface plasmon array, a fluorescence microscope, a Raman spectroscope, an optical detector in combination with a light source or a combination of same.

8. The device (10, 40) according to any one of claims 2 to 7, wherein the sensor system comprises an impedance meter.

9. The device (10, 40) according to any one of claims 2 to 8, wherein the sensor system comprises an electrochemical microelectrode, which is electrically coupled to the receptor cell (14) and is implemented to detect the electric potential of the receptor cell (16).

10. The device (10, 40) according to any one of claims 1 to 9, wherein the receptor cell (14) has an indicator dye or a marker dye or colored/fluorescent aptamers, peptides, proteins, antibodies, antigens, DNA and/or RNA, which comprises a fluorescence characteristic that depends on the cellular state, and
wherein the sensor system (16) comprises an optical sensor, which is implemented to detect the fluorescence characteristic or the UV-Vis absorption or the Raman or IR or SPR characteristic of the receptor cell (14).

11. The device (10, 40) according to any one of claims 1 to 10, having a substrate (22, 42) and an artificial epithelium (20), which is arranged on an area of the substrate (22, 42), wherein the receptor cell (14) is formed on the artificial epithelium (20) in order to be supplied with nutrients by means of same.

12. The device (10, 40) according to claim 11, having a sensor (44, 46) for determining an ambient condition of the epithelium (20).

13. The device (10, 40) according to any one of claims 1 to 12, having an analyte receptacle (48) which is effectively connected to the receptor cell (14) to enable a supply of the analyte (12) to the receptor cell (14).

14. The device (10, 40) according to any one of claims 1 to 13, in which the receptor cell (14) is implemented to react selectively with a change in cellular state to a type group of the analyte (12).

15. The device (10, 40) according to any one of claims 1 to 14, having a plurality of different organic receptor cells (14), each being designed to selectively respond to different type groups of the analyte (12) with a change in cell state.

16. The device (10, 40) according to any one of claims 1 to 15, wherein the sensory perception comprises the sense of taste, and the receptor cell (14) comprises a taste bud.

17. The device (10, 40) according to any one of claims 1 to 16, wherein the sensory perception is a human or animal sensory perception.

18. A system for detecting a sensory perception with regard to an analyte (12), comprising
a device (10, 40) according to any one of claims 1 to 17; and
a signal analyzer designed to determine the sensory perception with regard to the analyte (12) on the basis of the cellular state signal (18).

19. The system according to Claim 18, wherein the sensor perception is determined by comparing the cellular state signal (18) with a previously determined cellular state signal (18) of a known analyte (12).

20. A method for detecting a sensory perception with regard to an analyte (12) comprising:
providing the analyte (12);
supplying the analyte (12) to an organic receptor cell (14) for detection of a sensory perception;
determining the cell state of the receptor cell (14) which depends on the analyte (12) attached to the receptor cell (14) by detecting a fluorescence characteristic that depends on the cellular state or a UV-Vis or Raman or IR or SPR characteristic of a nanoparticle (28) attached to the organic receptor cell (14); and
output of a cellular state signal (18) indicating the cellular state.

21. The method according to claim 20, further comprising:
determining the sensor perception with regard to the analyte (12) on the basis of the cellular state signal (18).
